# EUROPÄISCHE PATENTANMELDUNG

(11) **EP 0 837 064 A2**
(43) Veröffentlichungstag der Anmeldung: **22.04.1998**
(21) Anmeldenummer: 97810747.2
(22) Anmeldetag: 07.10.1997
(51) Int. Cl.: C07D 405/06, C07D 405/12, C08K 5/3435, C08L 71/02

(54) **Addukte aus Aminen und Epoxyd-HALS und ihre Verwendung als Stabilisatoren**

(30) Priorität: 16.10.1996 CH 2523/96
(71) Anmelder: Ciba Specialty Chemicals Holding Inc., 4057 Basel (CH)
(72) Erfinder: Steinmann, Alfred, 1724 Praroman (CH)

(57) **Zusammenfassung**

Es werden Verbindungen beschrieben, erhältlich durch Umsetzung von Verbindungen der allgemeinen Formeln 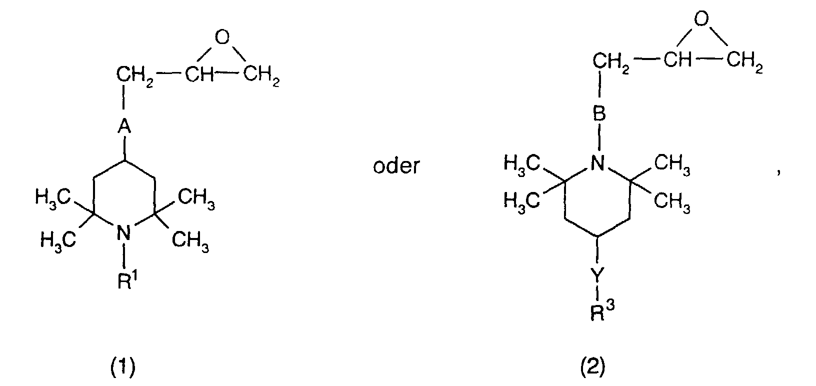 wobei
A gleich O oder N-R² ist und
B eine direkte Bindung oder O-CH₂-CH₂ darstellt, wobei der Kohlenstoff der Ethylengruppe mit dem Stickstoff des Piperidinringes verbunden ist, und R¹ H, C₁-C₂₀-Alkyl, C₂-C₂₀-Alkenyl, C₂-C₂₀-Alkinyl, C₆-C₂₀-Aryl, C₇-C₂₀-Aralkyl, O-C₁-C₂₀-Alkyl, O-C₅-C₈-Cycloalkyl, CO-C₁-C₂₀-Alkyl, CO-C₆-C₂₀-Aryl, CO-C₇-C₂₀-Aralkyl, O-CO-C₁-C₂₀-Alkyl oder C₁-C₆-Alkyl-Z-C₁-C₆-Alkyl darstellt, wobei Z O, S oder C=O ist und R² C₁-C₁₂-Alkyl oder 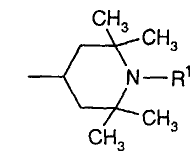 darstellt und Y O oder N-R⁴ ist, oder Y-R³ nach dem Entfernen des Wasserstoffs in 4-Stellung des Piperidinringes den divalenten Rest 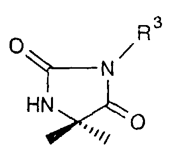 [→ Spiro-Verbindung] darstellt, und R³ C₁-C₂₀-Alkyl, CO-C₁-C₂₀-Alkyl, CO-C₆-C₂₀-Aryl oder CO-C₇-C₂₀-Aralkyl bedeuten, R⁴ C₁-C₂₀-Alkyl, oder, falls
R³ von C₁-C₂₀-Alkyl verschieden ist, auch Wasserstoff bedeutet,
mit mindestens einem sekundären oder primären Amin oder Ammoniak.

## Beschreibung

Die Erfindung betrifft neue Verbindungen, die durch Umsetzung epoxidgruppenhaltiger gehinderter Amine [HALS] mit primären oder sekundären Aminen oder Ammoniak erhalten werden können, deren Verwendung als Stabilisatoren organischen Materials gegen den schädigenden Einfluss von Licht, Sauerstoff und/oder Wärme, sowie die entsprechenden stabilisierten Zusammensetzungen.

Die Herstellung einiger Verbindungen vom Typ 2,2,6,6-Tetramethyl-4-(2,3-epoxypropoxy)piperidin sowie ihre Verwendung als Stabilisatoren für organische Polymere wird beispielsweise durch Luston und Vass, Makromolekulare Chemie, Macromol. Symp. 27, 231 (1989), beschrieben.

Reaktionsprodukte dieser Epoxide mit Toluolsulfonsäure und Ethylendisulfonsäure, sowie deren kombinierte Verwendung als Härtungskatalysatoren und Lichtschutzmittel in Lacken sind in der EP-A-0 097 616 genannt.

Die Bindung reaktiver Alkylpiperidine an Fluorpolymere, die freie Carboxygruppen enthalten, wird in EP-A-526 399 beschrieben.

Die Publikation EP-A-001 835 lehrt die weitere Umsetzung der epoxidgruppenhaltigen Piperidine mit Dicarbonsäureanhydriden zu Polyestern.

Die US-A-5,457,204 beschreibt die Umsetzung von Tetramethyl-4-(2,3-epoxy-propoxy)piperidinen mit Carbonsäuren oder Phenolen zu Esteralkoholen bzw. Hydroxyphenolethern und deren Verwendung als Stabilisatoren.

Es besteht weiterhin Bedarf an neuen Stabilisatoren vom Typ der 2,2,6,6-Tetraalkylpiperidine mit verbesserten Gebrauchseigenschaften, insbesondere besserer thermischer und hydrolytischer Beständigkeit als die der in US-A-5,457,204 genannten Verbindungen.

Es wurde nun gefunden, dass bestimmte Verbindungen, ausgehend vom Typ der Epoxygruppen enthaltenden 2,2,6,6-Tetramethylpiperidine und beliebigen primären oder sekundären Aminen oder Ammoniak, sich überraschend gut als Stabilisatoren für organisches Material eignen. Sie weisen auch eine hohe Temperaturbeständigkeit auf und erlauben somit in der Anwendung höhere Verarbeitungstemperaturen und damit einen höheren Durchsatz bei der weiteren Verarbeitung der so stabilisierten Produkte.

Gegenstand der Erfindung sind daher Verbindungen, erhältlich durch Umsetzung von Verbindungen der allgemeinen Formeln 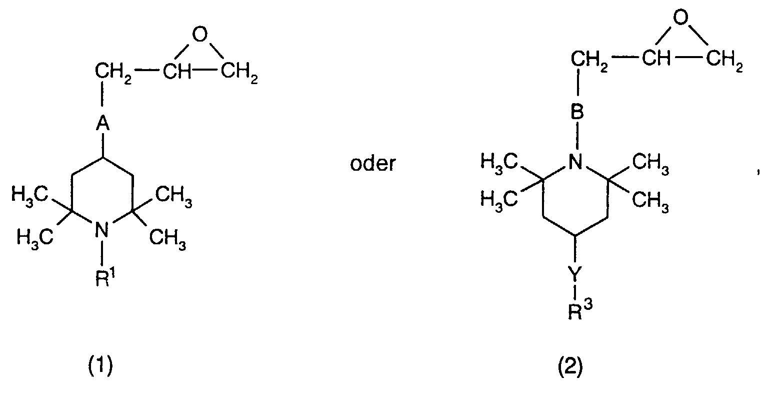 wobei
A gleich O oder N-R² ist und
B eine direkte Bindung oder O-CH₂-CH₂ darstellt, wobei der Kohlenstoff der Ethylengruppe mit dem Stickstoff des Piperidinringes verbunden ist, und
R¹ H, C₁-C₂₀-Alkyl, C₂-C₂₀-Alkenyl, C₂-C₂₀-Alkinyl, C₆-C₂₀-Aryl, C₇-C₂₀-Aralkyl, O-C₁-C₂₀-Alkyl, O-C₅-C₈-Cycloalkyl, CO-C₁-C₂₀-Alkyl, CO-C₆-C₂₀-Aryl, CO-C₇-C₂₀-Aralkyl, O-CO-C₁-C₂₀-Alkyl oder C₁-C₆-Alkyl-Z-C₁-C₆-Alkyl darstellt, wobei
Z O, S oder C=O ist und
R² C₁-C₁₂-Alkyl oder 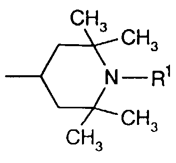 darstellt und
Y O oder N-R⁴ ist, oder Y-R³ nach dem Entfernen des Wasserstoffs in 4-Stellung des Piperidinringes den divalenten Rest 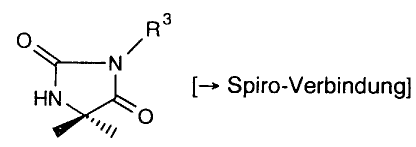 darstellt, und
R³ C₁-C₂₀-Alkyl, CO-C₁-C₂₀-Alkyl, CO-C₆-C₂₀-Aryl oder CO-C₇-C₂₀-Aralkyl bedeuten,
R⁴ C₁-C₂₀-Alkyl, oder, falls
R³ von C₁-C₂₀-Alkyl verschieden ist, auch Wasserstoff bedeutet,
mit mindestens einem sekundären oder primären Amin oder Ammoniak.

R¹, R², R³ und R⁴ als C₁-C₂₀-Alkyl [R² bis C₁₂] bedeuten z. B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl u.s.w.
Die Alkylgruppen R¹, R², R³ und R⁴ weisen insbesondere 1 bis 12, vorzugsweise 1 bis 8 C-Atome auf.

R¹ als C₅-C₈-Cycloalkyl bedeutet Cyclopentyl, Cyclohexyl, Cycloheptyl und Cyclooctyl. Bevorzugt ist Cyclohexyl.

R¹ als C₂-C₂₀-Alkenyl bedeutet z. B. Vinyl, 1-Propenyl, 2-Propenyl, 1-Butenyl, 2-Butenyl, 3-Butenyl u.s.w. Bevorzugt sind Alkenylgruppen mit 2 bis 12 C-Atomen, insbesondere mit 2 bis 8 C-Atomen.

R¹ als C₂-C₂₀-Alkinyl bedeutet z. B. Ethinyl, 1-Propinyl, 2-Propinyl, 1-Butinyl, 2-Butinyl, 3-Butinyl u.s.w. Bevorzugt haben solche Alkinylgruppen 2 bis 12 C-Atome, besonders bevorzugt sind solche mit 2 bis 8 C-Atomen, insbesondere 3 C-Atome.

R¹ und R³ als C₆-C₂₀-Aryl bedeuten z. B. Phenyl, Naphthyl, Anthracenyl, Phenanthrenyl. Bevorzugt ist Phenyl.

R¹ und R³ als Aralkyl bedeuten z. B. Benzyl, Phenethyl, 3-Phenylpropyl, α-Methylbenzyl oder a,a'-Dimethylbenzyl.
Bevorzugt ist Benzyl.

Die Aminkomponenten werden ausgewählt aus den Verbindungsklassen der Piperidine, Alkylamine, Polyalkylenpolyamine, Polyaminoamide, Polyoxyalkylenpolyamine, aromatischen Amine oder nukleophile Aminogruppen enthaltendes Triazin, Aminocarbonsäuren oder Ammoniak, wobei Piperidine, die mindestens zwei Stickstoffatome enthalten als auch Polyoxyalkylenpolyamine, die aminterminiertes Polyethylenglykol oder Polypropylenglykol oder beide Polyalkylenglykole beinhalten, aromatische Amine mit höchstens 2 aromatischen Kohlenstoffringen, an den C-Atomen mit Aminogruppen oder Aminogruppen enthaltenden Substituenten substituiertes Triazin, aromatische Aminocarbonsäuren oder Ammoniak bevorzugt sind.

Bevorzugt sind Piperidine der Formel 3 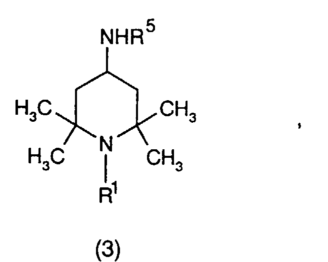 wobei
R¹ H, C₁-C₂₀-Alkyl, C₂-C₂₀-Alkenyl, C₂-C₂₀-Alkinyl, C₆-C₂₀-Aryl, C₇-C₂₀-Aralkyl, O-C₁-C₂₀-Alkyl, O-C₅-C₈-Cydoalkyl, CO-C₁-C₂₀-Alkyl, CO-C₆-C₂₀-Aryl, CO-C₇-C₂₀-Aralkyl, O-CO-C₁-C₂₀-Alkyl oder C₁-C₆-Alkyl-Z-C₁-C₆-Alkyl ist, wobei
Z O, S oder C=O ist und
R⁵ H, C₁-C₁₂-Alkyl oder 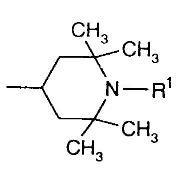 ist;
oder Alkylamine oder Dialkylamine, die maximal 20 C-Atome enthalten; Polyamine der Formeln 4 bis 6 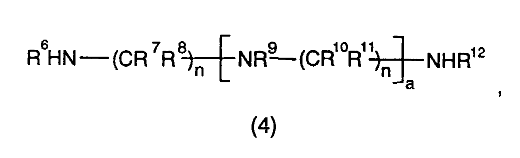 wobei
R⁶ bis R¹² unabhängig voneinander H, CH₃, C₂H₅ oder C₃H₇ sind, und
R⁹ zusätzlich R⁶HN-(CR⁷R⁸)ₙ bedeuten kann,
n 2 bis 20 ist und
a 0 bis 30 ist, 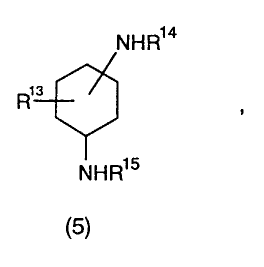 wobei
R¹³ bis R¹⁵ unabhängig voneinander H, CH₃ oder 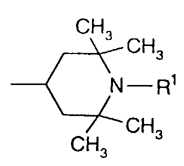 sind, oder 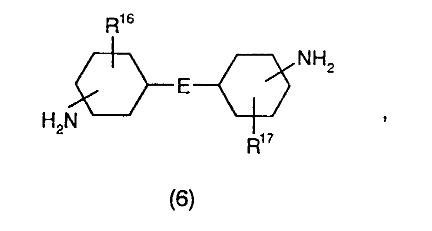 wobei
E eine direkte Bindung oder CH₂, C(CH₃)₂, C=O, N-H, S, SO, SO₂ oder O ist und
R¹⁶ und R¹⁷ unabhängig voneinander H, CH₃ oder 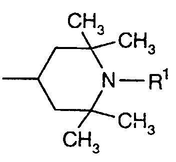 sind;

Polyaminoamide der Formel 7 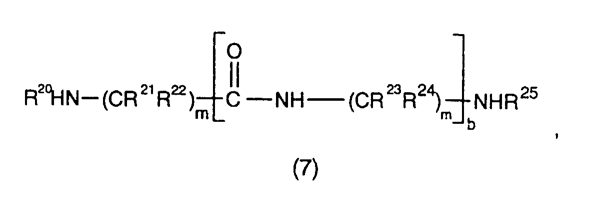 wobei
R²⁰ bis R²⁵ unabhängig voneinander H oder CH₃ sind,
m 1 bis 20 ist und
b 1 bis 30 ist;

Polyalkylenpolyamine oder Polyoxyalkylenpolyamine der Formel 8 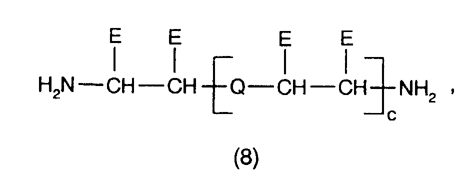 wobei die Symbole
E unabhängig voneinander H oder CH₃ bedeuten und
Q unabhängig voneinander O oder NH ist und
c 1 bis 10.000 ist;
aromatische Amine der Formel 9 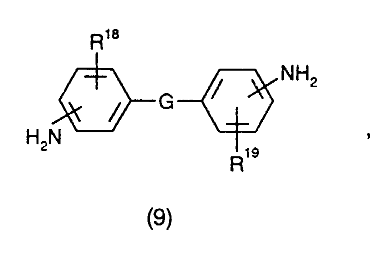 wobei
G eine direkte Bindung oder CH₂, C(CH₃)₂, C=O, N-H, S, SO, SO₂ oder O ist und
R¹⁸ und R¹⁹ unabhängig voneinander H, CH₃ oder C₂H₅ sind;
substituierte Triazine der Formel 10 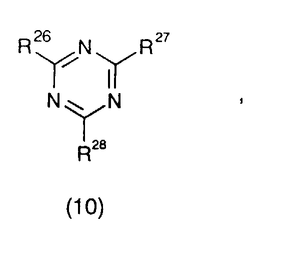 wobei
R²⁶ bis R²⁸ unabhängig voneinander einen primären und/oder sekundären Aminrest enthaltenden Substituenten mit einer C-Zahl von 1 bis 18 darstellen;
   Aminocarbonsäuren der Formel 11 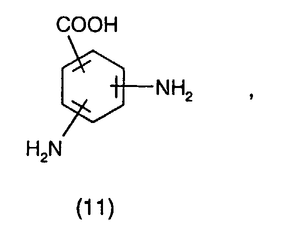 oder Ammoniak.
R¹ und R⁵ der Formel 3 als C₁-C₂₀-Alkyl haben die gleiche Bedeutung wie R¹, R³ und R⁴ als C₁-C₂₀-Alkyl der Epoxidverbindungen der Formeln 1 und 2.

Alkylamine oder Dialkylamine, die maximal 20 C-Atome enthalten, bedeuten z. B. Methylamin, Ethylamin, n-Propylamin, Isopropylamin, n-Butylamin, Isobutylamin, tert.-Butylamin, n-Pentylamin, 2-Pentylamin, 3-Pentylamin, Pentaerythrylamin u.s.w. oder Dimethylamin, Diethylamin, Di-n-propylamin, Diisopropylamin, Di-n-butylamin, Diisobutylamin, Di-tert.-butylamin, Di-n-pentylamin, Di-2-pentylamin, Di-3-pentylamin, Di-pentaerythrylamin u.s.w. , d. h. Amine, deren C-Atomketten linear als auch verzweigt sein können. Es können auch Dialkylamine eingesetzt werden, die unterschiedliche Alkylketten, sowohl linear als auch verzweigt, enthalten, wie z. B. Methyl-isopropylamin.

Bei den Polyaminen der Formel 4 handelt es sich um Polyamine wie beispielsweise Methylendiamin, 1,4-Butylendiamin, 2-Methylpentamethylendiamin, Hexamethylendiamin (HMD), Trimethylhexamethylendiamin (TMD), Bis-hexamethylentriamin (BHMD) oder Ethylendiamin (EDA), Diethylentriamin (DETA), Triethylentetramin (TETA), Tetraethylenpentamin (TEPA), Pentaethylenhexamin u.s.w. oder um 1,3-Propylendiamin, 1,2-Propylendiamin, Dipropylentriamin, Tripropylentetramin oder Tris(2-aminoethyl)amin, u.s.w. als auch um Amine, deren Alkylenketten verzweigt sind wie im Diisopropylentriamin. Die terminierenden Stickstoffatome können ebenso einen Alkylsubstituenten aus der Gruppe Methyl, Ethyl, n-Propyl oder Isopropyl tragen.
Ebenso sind Polyamine einsetzbar, welche cyclische Ringe beinhalten. Diese Ringe können sowohl aromatisch als auch cycloaliphatisch sein.

Typische einkernige aromatische Diamine sind beispielsweise die Xylylendiamine, wie z. B. m-Xylylendiamin (MXDA).

Typische Vertreter der cycloaliphatischen Diamine der Formel 5 sind das 1 ,2-Diaminocyclohexan (1,2-DACH) oder dessen Isomere bzw. das 1 -Methyl-2,4-diaminocyclohexan.

Isophorondiamin (IPD), 1,3-Bis-aminomethylcyclohexan (1,3-BAC) und 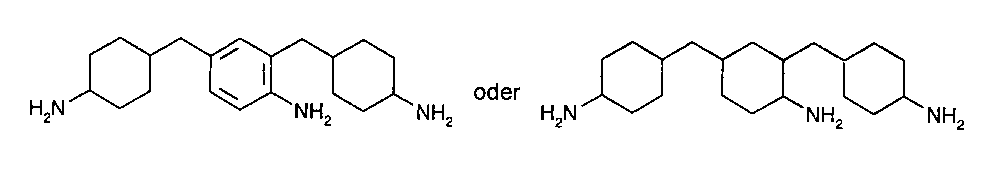 sind weitere einsetzbare cycloaliphatische Polyamine.

Beispiele für Polyamine der Formel 6 sind: 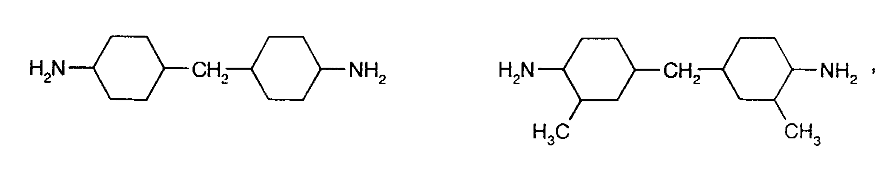 wobei das 4,4'-Diaminodicyclohexylmethan bevorzugt ist.

Norbornandiamin als auch N-2-Aminoethylpiperazin sind weitere typische Vertreter für Polyamine.

Polyaminoamide der Formel 7 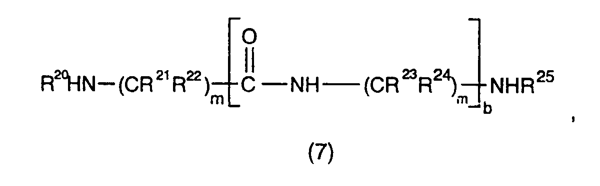 enthalten bis zu 30 wiederkehrende Amido-Einheiten, wobei die Kohlenstoffkette verzweigt sein kann.
Polyaminoamide können ebenso das Umsetzungsprodukt aus einer gegebenenfalls ungesättigten Fettsäure und einem Polyamin sein oder z. B. das Produkt aus Adipinsäure und Diethylentriamin, d. h. das Umsetzungsprodukt aus einer Dicarbonsäure und einem Polyamin.

Zu den Polyoxyalkylenpolyaminen der Formel 8 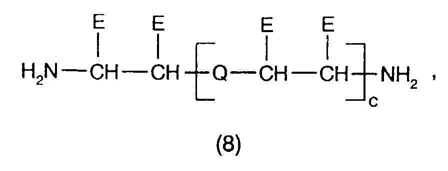 die für die Zwecke der vorliegenden Erfindung geeignet sind, gehören z. B. die Jeffamine® der Texaco Chemical Corporation [Texaco-Broschüre, Publikations-Nr. SC-024, 102-0548].

Beispiele für aromatische Amine der Formel 9 sind 4,4'-Diaminodiphenylmethan (4,4'-DDM), 4,4'-Diaminodiphenylsulfon (4,4'-DDS), 4,4'-Diaminodiphenylether, 3,3'-Dimethyl-4,4'-diaminodiphenylmethan, 3,3'-Diethyl-4,4'-diaminodiphenylmethan u.s.w., wobei das 4,4'-DDM, das 4,4'-DDS und der 4,4'-Diaminodiphenylether bevorzugt sind.

Aminocarbonsäuren können sowohl aliphatischer, cycloaliphatischer als auch aromatischer Natur sein; es können ebenso alle drei Strukturtypen in einem Molekül auftreten. Die aliphatischen Strukturen können sowohl geradkettig als auch verzweigt sein. Heteroatome können ebenso eingebaut sein.
Ein Beispiel für eine aromatische Aminocarbonsäure ist z. B. die 3,5-Diaminobenzoesäure.

Für alle genannten Verbindungen gilt, dass alle möglichen Isomeren eingesetzt werden können.

Zur Herstellung der Ausgangsverbindungen der Formeln 1 bzw. 2 geht man beispielsweise von kommerziell erhältlichem 4-Hydroxy-2,2,6,6-tetramethylpiperidin oder 4-Alkylamino-2,2,6,6-tetramethylpiperidin bzw. 4-Alkoxy-2,2,6,6-tetramethylpiperidin, das aus 4-Hydroxy-2,2,6,6-tetramethylpiperidin und dem entsprechenden Alkylhalogenid herstellbar ist, aus und setzt diese in an sich bekannter Weise mit Epichlorhydrin um. Die Herstellung der Epoxide der Formeln 1 und 2 kann gemäss einer der Methoden erfolgen, die in der EP-A-0 634 399, EP-A-0 001 835 oder bei Luston und Vass, Makromolekulare Chemie, Macromol. Symp. 27, 231 (1989), beschrieben sind. Zweckmässig wird die Piperidinverbindung in Gegenwart starker Basen, beispielsweise wässriger konzentrierter Alkalihydroxidlösung, und gegebenenfalls eines organischen Lösungsmittels langsam mit einem Überschuss Epichlorhydrin versetzt.

Vorteilhaft wird die Base in ca. 2 bis 20 fachem molarem Überschuss bezogen auf die Ausgangsverbindung eingesetzt; beispielsweise werden 3 bis 15 Mol, bevorzugt 4 bis 12 Mol Natrium- oder Kaliumhydroxid als 50 % wässrige Lösung pro Mol Piperidinverbindung verwendet. Die Menge des organischen Lösemittels wird zweckmässig so bemessen, dass die Epoxidverbindung vollständig gelöst wird.
Die Reaktion erfolgt zweckmäßig in einem inerten Lösungsmittel. Als Lösungsmittel können polare oder unpolare organische Lösungsmittel eingesetzt werden wie z. B.
Kohlenwasserstoffe, Ether, Ketone, Amide, Nitrile, tert. Amine oder Sulfoxide; geeignet sind beispielsweise Toluol, Hexan, Cyclohexan, Ligroin, Petrolether und andere Kohlenwasserstoffgemische, Dimethylformamid, Tetrahydrofuran, Dioxan, Diethylether, Dimethylsulfoxid und Acetonitril; besonders bevorzugt ist Toluol.
Auf ein Äquivalent der Piperidinausgangsverbindung können beispielsweise 1 bis 4, vorzugsweise 1,2 bis 3, vor allem 1,5 bis 2,5 Äquivalente Epichlorhydrin eingesetzt werden. Darüber hinaus können der Mischung vorteilhaft 0,01 bis 10 Mol-%, bevorzugt 2 bis 4 Mol-%, eines tert. Aminsalzes, beispielsweise eines Tetraalkylammoniumhalogenids wie Tetramethylammoniumchlorid oder Tetrabutylammoniumbromid, oder eines Phosphoniumsalzes, beispielsweise eines quartären Phosphoniumhalogenids wie Ethyltriphenylphosphoniumbromid, als Katalysator zugesetzt werden.
Die Temperatur kann während der Reaktion im Bereich von 0 bis 100°C liegen, zweckmässig beträgt die Temperatur 30 bis 80°C, vor allem 40 bis 70°C.

Die Herstellung der erfindungsgemässen Amino-Addukte erfolgt dann in einer zweiten Reaktion zweckmässig ohne Lösungsmittel und bei erhöhter Temperatur.
Die Temperatur kann während der Reaktion im Bereich von 80 bis 250°C liegen, zweckmässig beträgt die Temperatur 100 bis 200°C, vor allem 170 bis 190°C.
Bei Verbindungen mit R¹ = O-C₁-C₂₀-Alkyl, O-C₅-C₈-Cycloalkyl oder O-CO-C₁-C₂₀-Alkyl liegt die Reaktionstemperatur unter 100°C.

Bei der Reaktion der Verbindungen der Formel 1 bzw. 2 mit primären oder sekundären Aminen, die mehr als einen reaktionsfähigen Stickstoff enthalten, können alle oder auch nur einige oder eine der Aminofunktionen mit dem eine Epoxidfunktion enthaltenden Piperidin abreagieren.
Ebenso können für die Reaktion Mischungen von zwei oder mehr Aminen eingesetzt werden, die im obigen Sinne abreagieren können.

Falls die Reaktion in einem inerten Lösungsmittel ausgeführt wird, so kann die Temperatur der Reaktionsmischung für die Dauer der Reaktion im Siedebereich gehalten werden (Rückfluß). Dazu wird eine Lösemittel enthaltende Reaktionsmischung, im allgemeinen unter Normaldruck, zum Siedepunkt erwärmt und das verdampfte Lösemittel mit Hilfe eines geeigneten Kühlers kondensiert und wieder der Reaktionsmischung zugeführt.

Die Reaktionen können unter Ausschluß von Sauerstoff durchgeführt werden, beispielsweise durch Spülen mit einem Inertgas wie Argon; Sauerstoff stört jedoch nicht in jedem Fall, so daß die Reaktion auch ohne die genannte Maßnahme durchgeführt werden kann.

Nach beendeter Reaktion kann die Aufarbeitung nach gängigen Methoden erfolgen; zweckmäßig wird die Mischung zunächst mit Wasser verdünnt, beispielsweise indem das Reaktionsgemisch in das 1-4-fache Volumen (Eis-)Wasser gegeben wird; anschließend kann das Produkt direkt abgetrennt oder extrahiert werden, zur Extraktion eignet sich z. B. Essigester oder Toluol. Wird extrahiert, so kann das Produkt in üblicher Weise durch Entfernen des Lösemittels isoliert werden; dies geschieht zweckmäßig nach Trocknen der organischen Phase. Möglich ist auch das Einschalten weiterer Reinigungsschritte wie beispielsweise Waschen mit wässriger Natriumbicarbonatlösung, Dispergieren von Aktivkohle, chromatographieren mittels Kieselgels, Filtrieren, Umkristallisieren und/oder Destillieren.

Bei der Reaktion der Verbindungen der Formeln 1 oder 2 mit primären oder sekundären Aminen oder Ammoniak entstehen die erfindungsgemässen Verbindungen, die folgende Strukturelemente I und II enthalten: 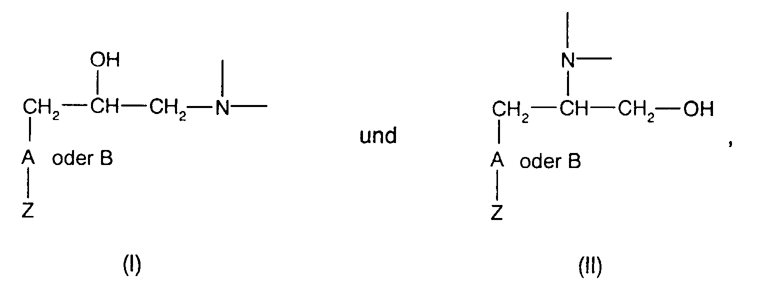 wobei Z das Symbol für den Piperidin-Rest darstellt und A und B die auf Seite 2 und in Anspruch 1 gegebene Definition haben. Die Verbindungen der Formel I werden dabei bevorzugt gebildet und stellen somit das Hauptprodukt dar.

Die Verbindungen gemäss Anspruch 1 eignen sich besonders zum Stabilisieren von organischen Materialien gegen thermischen, oxidativen und aktinischen Abbau.

Beispiele für derartige Materialien sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z. B. von Cyclopenten oder Norbornen; ferner Polyethylen (das gegebenenfalls vernetzt sein kann), z. B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d. h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler(-Natta), TNZ (DuPont), Metallocen oder Single Site Katalysatoren (SSC) bezeichnet.
2. Mischungen der unter 1) genannten Polymeren, z. B. Mischungen von Polypropylen mit Polyisobutylen, Polypropylen mit Polyethylen (z. B. PP/HDPE, PP/LDPE) und Mischungen verschiedener Polyethylentypen (z. B. LDPE/HDPE).
3. Copolymere von Mono- und Diolefinen untereinander oder mit anderen Vinylmonomeren, wie z. B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z. B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z. B. Polyamiden.
4. Kohlenwasserstoffharze (z. B. C₅-C₉) inklusive hydrierte Modifikationen davon (z. B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Butadien-Alkylacrylat und - methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z. B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z. B. als sogenannte ABS-, MBS-, ASA- oder AES-Polymere bekannt sind.
8. Halogenhaltige Polymere, wie z. B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z. B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z. B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z. B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z. B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von Polyepoxiden ableiten, z. B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z. B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend a) ein gegen oxidativen, thermischen oder/und aktinischen Abbau/Aufbau empfindliches organisches Material und b) mindestens eine Verbindung gemäss Anspruch 1, sowie die Verwendung von Verbindungen gemäss Anspruch 1 zum Stabilisieren von organischem Material gegen oxidativen, thermischen oder aktinischen Abbau/Aufbau. Die Erfindung umfaßt ebenfalls ein Verfahren zum Stabilisieren von organischem Material gegen thermischen, oxidativen oder/und aktinischen Abbau/Aufbau, dadurch gekennzeichnet, daß man diesem Material mindestens eine Verbindung gemäss Anspruch 1 zusetzt.

Von besonderem Interesse ist die Verwendung von Verbindungen gemäss Anspruch 1 als Stabilisatoren in synthetischen organischen Polymeren sowie entsprechende Zusammensetzungen.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyolefine, vor allem Polyethylen (PE) und Polypropylen (PP). Ebenfalls besonders bevorzugte organische Materialien sind Überzugszusammensetzungen. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A 18, Seiten 359-464,
VCH Verlagsgesellschaft, Weinheim 1991.

Von besonderem Interesse ist die Verwendung der erfindungsgemäßen Verbindungen als Stabilisatoren für Überzüge, beispielsweise für Lacke. Gegenstand der Erfindung sind daher auch solche Zusammensetzungen, deren Komponente A ein filmbildendes Bindemittel ist.

Das erfindungsgemäße Überzugsmittel enthält vorzugsweise auf 100 Gew.-Teile festes Bindemittel A 0,01-10 Gew.-Teile, insbesondere 0,05-10 Gew.-Teile, vor allem 0,1-5 Gew.-Teile des erfindungsgemäßen Stabilisators B.

Auch hier sind Mehrschichtsysteme möglich, wobei die Konzentration der Komponente B in der Deckschicht höher sein kann, beispielsweise 1 bis 15 Gew.-Teile, vor allem 3 bis 10 Gew.-Teile B auf 100 Gew.-Teile festes Bindemittel A.

Die Verwendung der erfindungsgemässen Verbindungen als Stabilisator in Überzügen bringt den zusätzlichen Vorteil mit sich, daß der Delaminierung, d. h. dem Abblättern des Überzuges vom Substrat, vorgebeugt wird. Dieser Vorteil kommt insbesondere bei metallischen Substraten zum tragen, auch bei Mehrschichtsystemen auf metallischen Substraten.

Als Bindemittel (Komponente A) kommen prinzipiell alle in der Technik gebräuchlichen in Betracht, beispielsweise solche, wie sie beschrieben sind in Ullmann's Encyclopedia of Industrial Chemistry, 5. Ed., Vol. A 18, Seiten 368-426, VCH Verlagsgesellschaft, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Komponente A kann ein kalt aushärtbares oder ein heiß aushärtbares Bindemittel sein, wobei die Zugabe eines Härtungskatalysators vorteilhaft sein kann. Geeignete Katalysatoren, die die Aushärtung des Bindemittels beschleunigen, sind beispielsweise beschrieben in Ullmann's Encyclopedia of Industrial Chemistry, Vol. A 18, S. 469, VCH Verlagsgesellschaft, Weinheim 1991.

Bevorzugt sind Überzugsmittel, worin die Komponente A ein Bindemittel aus einem funktionellen Acrylatharz und einem Vernetzer ist.

Beispiele von Überzugsmitteln mit speziellen Bindemitteln sind:
1. Lacke auf Basis von kalt- oder heiß-vernetzbaren Alkyd-, Acrylat-, Polyester-, Epoxid- oder Melaminharzen oder Mischungen solcher Harze, gegebenenfalls mit Zusatz eines Härtungskatalysators;
2. Zweikomponenten-Polyurethanlacke auf Basis von hydroxylgruppenhaltigen Acrylat-, Polyester- oder Polyetherharzen und aliphatischen oder aromatischen Isocyanaten, Isocyanuraten oder Polyisocyanaten;
3. Einkomponenten-Polyurethanlacke auf Basis von blockierten Isocyanaten, lsocyanuraten oder Polyisocyanaten, die während des Einbrennens deblockiert werden;
4. Einkomponenten-Polyurethanlacke auf Basis von aliphatischen oder aromatischen Urethanen oder Polyurethanen und hydroxylgruppenhaltigen Acrylat-, Polyester- oder Polyetherharzen;
5. Einkomponenten-Polyurethanlacke auf Basis von aliphatischen oder aromatischen Urethanen oder Polyurethanen mit freien Amingruppen in der Urethanstruktur und Melaminharzen oder Polyetherharzen, gegebenenfalls mit Zusatz eines Härtungskatalysators;
6. Zweikomponentenlacke auf Basis von (Poly)ketiminen und aliphatischen oder aromatischen Isocyanaten, lsocyanuraten oder Polyisocyanaten;
7. Zweikomponentenlacke auf Basis von (Poly)ketiminen und einem ungesättigten Acrylatharz oder einem Polyacetoacetatharz oder einem Methacrylamidoglykolatmethylester;
8. Zweikomponentenlacke auf Basis von carboxyl- oder aminogruppenhaltigen Polyacrylaten und Polyepoxiden;
9. Zweikomponentenlacke auf Basis von anhydridgruppenhaltigen Acrylatharzen und einer Polyhydroxy- oder Polyaminokomponente;
10. Zweikomponentenlacke auf Basis von acrylathaltigen Anhydriden und Polyepoxiden;
11. Zweikomponentenlacke auf Basis von (Poly)oxazolinen und anhydridgruppenhaltigen Acrylatharzen oder ungesättigten Acrylatharzen oder aliphatischen oder aromatischen Isocyanaten, lsocyanuraten oder Polyisocyanaten;
12. Zweikomponentenlacke auf Basis von ungesättigten Polyacrylaten und Polymalonaten;
13. thermoplastische Polyacrylatlacke auf Basis von thermoplastischen Acrylatharzen oder fremdvernetzenden Acrylatharzen in Kombination mit veretherten Melaminharzen;
14. Lacksysteme auf Basis von siloxanmodifizierten oder fluormodifizierten Acrylatharzen.

Das erfindungsgemäße Überzugsmittel enthält vorzugsweise neben den Komponenten A und B als Komponente C ein Lichtschutzmittel vom Typ der 2-Hydroxyphenyl-2H-benztriazole, beispielsweise wie sie in unten genannter Liste unter dem Punkt 2.1 aufgeführt sind. Von besonderem technischem Interesse ist dabei der Zusatz von 2-Hydroxyphenyl-2H-benztriazolen.

Die Komponente C wird vorzugsweise in einer Menge von 0,05-5 Gew.-Teilen auf 100 Gew.-Teile des festen Bindemittels verwendet.

Das Überzugsmittel kann außer den Komponenten A, B und gegebenenfalls C weitere Komponenten enthalten, z. B. Lösungsmittel, Pigmente, Farbstoffe, Weichmacher, Stabilisatoren, Thixotropiemittel, Trocknungskatalysatoren und/oder Verlaufhilfsmittel. Mögliche Komponenten sind beispielsweise solche, wie sie in Ullmann's Encyclopedia of Industrial Chemistry, 5. Ed., Vol. A 18, Seiten 429-471, VCH Verlagsgesellschaft, Weinheim 1991 beschrieben sind.

Mögliche Trocknungskatalysatoren beziehungsweise Härtungskatalysatoren sind beispielsweise organische Metallverbindungen, Amine, aminogruppenhaltige Harze oder/und Phosphine. Organische Metallverbindungen sind z. B. Metallcarboxylate, insbesondere solche der Metalle Pb, Mn, Co, Zn, Zr oder Cu, oder Metallchelate, insbesondere solche der Metalle Al, Ti oder Zr oder Organometallverbindungen wie z. B. Organozinnverbindungen.

Beispiele für Metallcarboxylate sind die Stearate von Pb, Mn oder Zn, die Octoate von Co, Zn oder Cu, die Naphthenate von Mn und Co oder die entsprechenden Linoleate, Resinate oder Tallate.

Beispiele für Metallchelate sind die Aluminium-, Titan- oder Zirkonium-Chelate von Acetylaceton, Ethylacetylacetat, Salicylaldehyd, Salicylaldoxim, o-Hydroxyacetophenon oder Ethyl-trifluoracetylacetat und die Alkoxide dieser Metalle.

Beispiele für Organozinnverbindungen sind Dibutylzinnoxid, Dibutylzinn-dilaurat oder Dibutylzinn-dioctoat.

Beispiele für Amine sind vor allem tertiäre Amine, wie z. B. Tributylamin, Triethanolamin, N-Methyl-diethanolamin, N-Dimethylethanolamin, N-Ethylmorpholin, N-Methylmorpholin oder Diazabicyclooctan (Triethylendiamin) sowie deren Salze. Weitere Beispiele sind quaternäre Ammoniumsalze, wie z. B. Trimethylbenzylammoniumchlorid.

Aminogruppenhaltige Harze sind gleichzeitig Bindemittel und Härtungskatalysator. Beispiel hierfür sind aminogruppenhaltige Acrylat-copolymere.

Als Härtungskatalysator können auch Phosphine verwendet werden, wie z. B. Triphenylphosphin.

Bei den erfindungsgemäßen Uberzugsmitteln kann es sich auch um strahlenhärtbare Überzugsmittel handeln. In diesem Fall besteht das Bindemittel im wesentlichen aus monomeren oder oligomeren Verbindungen mit ethylenisch ungesättigten Bindungen (Präpolymeren), die nach der Applikation durch aktinische Strahlung gehärtet, d. h. in eine vernetzte, hochmolekulare Form überführt werden. Handelt es sich um ein UV-härtendes System, enthält dies in der Regel zusätzlich einen Photoinitiator. Entsprechende Systeme sind in der oben genannten Publikation, Ullmann's Encyclopedia of Industrial Chemistry, 5. Ed., Vol. A 18, Seiten 451-453, beschrieben. In strahlenhärtbaren Überzugsmitteln können die erfindungsgemäßen Stabilisatoren auch ohne Zusatz von sterisch gehinderten Aminen eingesetzt werden.

Die erfindungsgemäßen Überzugsmittel können auf beliebige Substrate aufgebracht werden, beispielsweise auf Metall, Holz, Kunststoff oder keramische Materialien. Vorzugsweise werden sie beim Lackieren von Automobilen als Decklack verwendet. Besteht der Decklack aus zwei Schichten, wovon die untere Schicht pigmentiert ist und die obere Schicht nicht pigmentiert ist, so kann das erfindungsgemäße Überzugsmittel für die obere oder die untere Schicht oder für beide Schichten verwendet werden, vorzugsweise jedoch für die obere Schicht.

Die erfindungsgemäßen Überzugsmittel können auf die Substrate nach den üblichen Verfahren aufgebracht werden, beispielsweise durch Streichen, Besprühen, Gießen, Tauchen oder Elektrophorese; s. a. Ullmann's Encyclopedia of Industrial Chemistry, 5. Ed., Vol. A 18, Seiten 491-500.

Die Härtung der Überzüge kann - je nach dem Bindemittelsystem - bei Raumtemperatur oder durch Erwärmen erfolgen. Vorzugsweise härtet man die Überzüge bei 50-150°C, Pulverlacke auch bei höheren Temperaturen.

Die erfindungsgemäß erhaltenen Überzüge weisen eine hervorragende Beständigkeit gegen schädigende Einflüsse von Licht, Sauerstoff und Wärme auf; insbesondere ist auf die gute Licht- und Witterungsbeständigkeit der so erhaltenen Überzüge, beispielsweise Lacke, hinzuweisen.

Gegenstand der Erfindung ist daher auch ein Überzug, besonders ein Lack, der durch Zusatz mindestens einer erfindungsgemässen Verbindung gegen schädigende Einflüsse von Licht, Sauerstoff und Wärme stabilisiert ist. Der Lack ist vorzugsweise ein Decklack für Automobile. Die Erfindung beinhaltet weiterhin ein Verfahren zum Stabilisieren eines Überzuges auf Basis organischer Polymere gegen Schädigung durch Licht, Sauerstoff und/oder Wärme, dadurch gekennzeichnet, daß man dem Überzugsmittel mindestens eine erfindungsgemässe Verbindung beimischt, sowie die Verwendung der erfindungsgemässen Verbindungen in Überzugsmitteln als Stabilisatoren gegen Schädigung durch Licht, Sauerstoff und/oder Wärme.

Die Überzugsmittel können ein organisches Lösungsmittel oder Lösungsmittelgemisch enthalten, in dem das Bindemittel löslich ist. Das Überzugsmittel kann aber auch eine wässrige Lösung oder Dispersion sein. Das Vehikel kann auch ein Gemisch eines organischen Lösungmittels und Wasser sein. Das Überzugsmittel kann auch ein feststoffreicher Lack (high solids Lack) sein oder kann lösungsmittelfrei sein (z. B. Pulverlack). Pulverlacke sind beispielsweise solche, wie sie in Ullmann's Encyclopedia of Industrial Chemistry, 5. Ed., A 18, Seiten 438-444 beschrieben sind. Der Pulverlack kann auch in Form eines Pulver-Slurry, d. h. einer Dispersion des Pulvers bevorzugt in Wasser vorliegen.

Die Pigmente können anorganische, organische oder metallische Pigmente sein. Vorzugsweise enthalten die erfindungsgemäßen Überzugsmittel keine Pigmente und werden als Klarlack verwendet.

Ebenfalls bevorzugt ist der Einsatz des Überzugsmittels als Decklack für Anwendungen in der Automobilindustrie, besonders als pigmentierte oder unpigmentierte Deckschicht des Lackes. Die Verwendung für darunter liegende Schichten ist jedoch auch möglich.

Weitere mit den erfindungsgemässen Verbindungen zu stabilisierende Materialien sind photographische Materialien. Unter solchen sind insbesondere solche zu verstehen, die in Research Disclosure 1990, 31429 (Seiten 474-480) für die photographische Reproduktion und andere Reproduktionstechniken beschrieben sind.

Allgemein werden die Verbindungen gemäss Anspruch 1 dem zu stabilisierenden Material in Mengen von 0,01 bis 10%, bevorzugt 0,01 bis 5%, insbesondere 0,01 bis 2%, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5%, vor allem 0,1 bis 0,5%.

Die Einarbeitung in die Materialien kann beispielsweise durch Einmischen oder Aufbringen der Verbindungen gemäss Anspruch 1 und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen gemäss Anspruch 1 in Polymere besteht in deren Zugabe vor, während oder unmittelbar nach der Polymerisation der entsprechenden Monomeren bzw. vor der Vernetzung. Die Verbindungen gemäss Anspruch 1 können dabei als solche, aber auch in encapsulierter Form (z.B in Wachsen, Ölen oder Polymeren) zugesetzt werden. Im Falle der Zugabe vor oder während der Polymerisation können die Verbindungen gemäss Anspruch 1 auch als Regulator für die Kettenlänge der Polymere (Kettenabbrecher) wirken.

Die Verbindungen gemäss Anspruch 1 können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der Verbindungen gemäss Anspruch 1 nach folgenden Methoden erfolgen:
- als Emulsion oder Dispersion (z. B. zu Latices oder Emulsionspolymeren),
- als Trockenmischung während des Vermischens von Zusatzkomponenten oder Polymermischungen,
- durch direktes Zugeben in die Verarbeitungsapparatur (z. B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z. B. als (zu) Folien, Fasern, Bändchen, Formmassen, Profilen, oder als Bindemittel für Lacke, Klebstoffe oder Kitte.

Neben den Verbindungen gemäss Anspruch 1 können die erfindungsgemäßen Zusammensetzungen als zusätzliche Komponente (c) ein oder mehrere herkömmliche Additive enthalten, wie beispielsweise die folgenden:
1. Antioxidantien
   1.1. Alkylierte Monophenole, z. B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(a-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z. B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z. B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z. B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
   1.4. Tocopherole, z. B. α-Tocopherol, β-Tocopherol, γ-Tocopherol, δ-Tocopherol und Mischungen davon (Vitamin E).
   1.5. Hydroxylierte Thiodiphenylether, z. B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.
   1.6. Alkyliden-Bisphenole, z. B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'--Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1 1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol--bis[3,3-bis(3'-tert-butyl-4'-hydroxyphenyl)-butyrat], Bis(3-tert-butyl-4-hydroxy-5-methylphenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
   1.7. O-, N- und S-Benzylverbindungen, z. B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis (3,5-di-tert-butyl-4-hydroxybenzyl)-sulfid, Isooctyl-3,5-di-tert-butyl-4-hydroxybenzyl-mercaptoacetat.
   1.8. Hydroxybenzylierte Malonate, z. B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.
   1.9. Hydroxybenzyl-Aromaten, z. B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.
   1.10. Triazinverbindungen, z. B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1 ,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.
   1.11. Benzylphosphonate, z. B. Dimethyl-2,5-di-tert-butyl-4-hydroxybenzylphosphonat, Di-ethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z. B. 4-Hydroxy-laurinsäureanilid, 4-Hydroxystearinsäureanilid, N-(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(5-tert-Butyl-4-hydroxy-3-methylphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicydohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1 -phospha-2,6,7-trioxabicyclo-[2.2.2]-octan.
   1.16. Ester der 3,5-Di-tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris (hydroxyethyl)-isocyanurat, N, N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.17. Amide der β-(3,5-Di-tert-butyl-4-hydroxyphenyl)-propionsäure, wie z. B. N,N'-Bis(3,5--di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis(3,5-di-tert-butyl-4-hydroxyphenylpropionyl)-hydrazin.
   1.18. Ascorbinsäure (Vitamin C).
   1.19. Aminische Antioxidantien, wie z. B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z. B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylaminomethyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/lsohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.
2. UV-Absorber und Lichtschutzmittel
   2.1. 2-(2'-Hydroxyphenyl)-benzotriazole, wie z. B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300;mit R = 3'-tert-Butyl-4'-hydroxy-5'-2H-benzotriazol-2-yl-phenyl.
   2.2. 2-Hydroxybenzophenone, wie z. B. das 4-Hydroxy-, 4-Methoxy-, 4-Octoxy-, 4-Decyloxy-,4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z. B. 4-tert-Butyl-phenyl-salicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z. B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyl dithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Weitere sterisch gehinderte Amine, wie z. B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4 -yl)-sebacat, n-Butyl-3,5-di-tert--butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1 -(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxospiro[4,5]decan, Umsetzungsprodukt von 7,7,9,9-Tetramethyl-2-cycloundecyl-1-oxa-3,8-diaza-4-oxospiro[4,5]decan und Epichlorhydrin.
   2.7. Oxalsäurediamide, wie z. B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o-und p-Methoxy- sowie von o- und p-Ethoxy-di-substituierten Oxaniliden.
   2.8. 2-(2-Hydroxyphenyl)-1,3,5-triazine, wie z. B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl) -4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4- (2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin.
3. Metalldesaktivatoren, wie z. B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z. B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis(2,4-di-tert-butyl-6-methylphenyl)-ethylphosphit.
5. Hydroxylamine wie z. B. N,N-Dibenzylhydroxylamin, N,N-diethylhydroxylamin, N,N-Di-octylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.
6. Nitrone wie z. B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecyl-nitron, N-Hexadecyl-alpha-pentadecyl-nitron, N-Octadecyl-alpha-heptadecyl-nitron, N-Hexadecyl-alpha-heptadecyl-nitron, N-Ocatadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosynergisten wie z. B. Thiodipropionsäure-di-laurylester oder Thiodipropionsäure-di-stearylester.
8. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.
9. Polyamidstabilisatoren, wie z. B. Kupfersalze in Kombination mit Jodiden und/oder Phosphorverbindungen und Salze des zweiwertigen Mangans.
10. Basische Co-Stabilisatoren, wie z. B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
11. Nukleierungsmittel*,* wie z. B. anorganische Stoffe wie z. B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z. B. 4-tert-Butylbenzoesäure, Adipinsäure, Diphenylessigsäure, Natriumsuccinat oder Natriumbenzoat; polymere Verbindungen wie z. B. ionische Copolymerisate ("Ionomere").
12. Füllstoffe und Verstärkungsmittel, wie z. B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.
13. Sonstige Zusätze, wie z. B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
14. Benzofuranone bzw. Indolinone, wie z. B. in US-A-4325863, US-A-4338244, US-A-5175312, US-A-5216052, US-A-5252643, DE-A-4316611 , DE-A-4316622, DE-A-431 6876, EP-A-0589839 oder EP-A-0591102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Diese zusätzlichen Additive werden zweckmäßig in Mengen von 0,1-10, beispielsweise 0,2-5 Gew.-%, bezogen auf das zu stabilisierende Polymer, eingesetzt.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen und der Tabelle werden die folgenden Abkürzungen verwendet:

| | |
|---|---|
| CDCl₃ | Deuterochloroform |
| Da | Dalton |
| DSC | Differential Scanning Calorimetry = Differentialthermoanalyse |
| GC | Gaschromatographie |
| ¹H-NMR | Magnetische Kernresonanz des Nuklids ¹H |
| MALDI - MS | Matrix Assisted Laser Desorption/lonization - Massenspektroskopie |
| mp | Schmelzpunkt |
| T_{g} | Glasübergangstemperatur |
| TGA | Thermogravimetrische Analyse - 10 %-iger Gewichtsverlust bei einer definierten Temperatur |

### Herstellungsbeispiele

### A1) Herstellung von 2,2,6,6-Tetramethyl-4-(2,3-epoxypropoxy)piperidin

In einem 750 ml Sulfierkolben mit mechanischem Rührer, Kühler und 100 ml Tropftrichter werden unter Argonatmosphäre 64,0 g (1,6 Mol) Natriumhydroxyd in 64 ml Wasser gelöst. Dazu gibt man 170 ml Toluol, 10,3 g (31,8 mMol) Tetrabutylammoniumbromid und 50 g (318 mMol) 4-Hydroxy-2,2,6,6-tetramethylpiperidin. Bei 45°C werden 58,8 g (636 mMol) Epichlorhydrin zugetropft. Man lässt dann bei 50°C während 4 Stunden rühren. Das Reaktionsgemisch wird auf Raumtemperatur abgekühlt und anschliessend auf 1 l Eiswasser gegossen, die organische Phase abgetrennt, diese mit Natriumsulfat getrocknet und am Rotationsverdampfer eingeengt. Der Rückstand wird bei 8-10⁻³ Torr destilliert. Siedepunkt: 48°C
Ausbeute: 28 g (41 % d. Th.), GC: 98 %

| Mikroanalyse: | | |
|---|---|---|
| | berechnet | gefunden |
| % C | 67,57 | 67,73 |
| % H | 10,87 | 10,92 |
| % N | 6,57 | 6,51 |

¹H-NMR (CDCl₃):

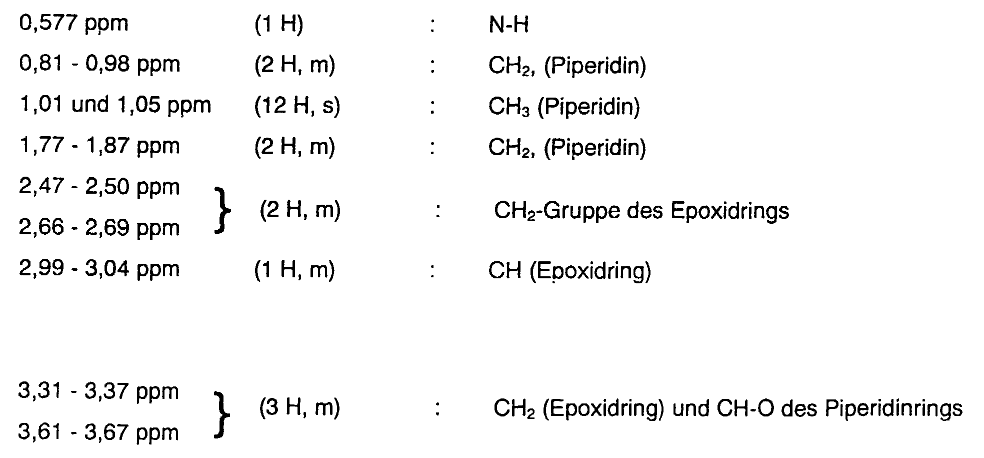

### A2) Herstellung von 1,2,2,6,6-Pentamethyl-4-(2,3-epoxypropoxy)piperidin

In einem 2,5 l Sulfierkolben mit mechanischem Rührer, Kühler und 500 ml Tropftrichter werden unter Argonatmosphäre 300 g (7,5 Mol) Natriumhydroxyd in 300 g Wasser gelöst. Dazu gibt man 750 ml Toluol, 48,4 g (0,15 Mol) Tetrabutylammoniumbromid und 257 g (1,5 Mol) 4-Hydroxy-1,2,2,6,6-pentamethylpiperidin. Bei 60°C werden 347 g (3,75 Mol) Epichlorhydrin in 1,5 Stunden zugetropft, anschliessend wird bei gleicher Temperatur für weitere 4 Stunden gerührt. Die Reaktionslösung wird auf 3 l Eiswasser gegossen, die organische Phase abgetrennt, diese mit Natriumsulfat getrocknet und eingedampft. Bei 0,05 Torr wird über eine Vigreuxkolonne destilliert und die Fraktion mit dem Siedepunkt 71 bis 72°C gesammelt.
Ausbeute: 205 g (60 % d. Th.), GC: > 99 %

| Mikroanalyse: | | |
|---|---|---|
| | berechnet | gefunden |
| % C | 68,68 | 68,64 |
| % H | 11,07 | 11,21 |
| % N | 6,16 | 6,32 |
| % Cl | 0,0 | 0,0 |

¹H-NMR (CDCl₃):

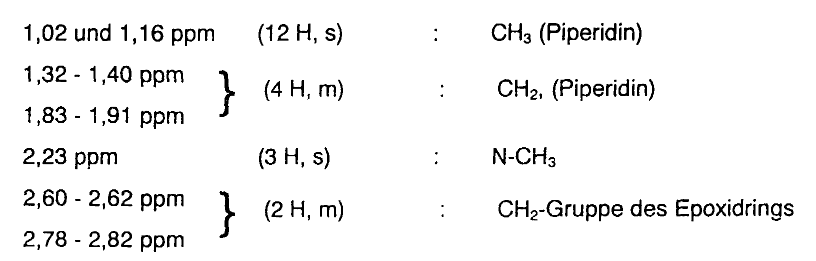

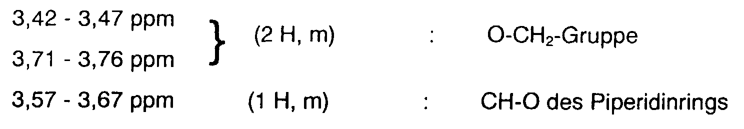

### B1) Addition von 1,2,2,6,6-Pentamethyl-4-(2,3-epoxypropoxy)piperidin an 4,4'-Diaminodiphenylmethan

In einem 250 ml Rundkolben, der mit einem Magnetrührer versehen ist, werden unter Argon 19,8 g (0,1 Mol) 4,4'-Diaminodiphenylmethan und 102,3 g (0,45 Mol) 1,2,2,6,6-Pentamethyl-4-(2,3-epoxypropoxy)piperidin vorgelegt. Die Mischung wird auf 180°C erwärmt und während 18 Stunden gerührt. Dann wird die klare Schmelze kurz im Hochvakuum von überschüssigem Epoxyd befreit und anschliessend auf Raumtemperatur abgekühlt. Man erhält 110 g (100 % d. Th.) eines farblosen, glasartigen Feststoffes.

| Mikroanalyse: | | |
|---|---|---|
| | berechnet | gefunden |
| % C | 70,48 | 70,32 |
| % H | 10,37 | 10,53 |
| % N | 7,59 | 7,48 |

¹H-NMR (CDCl₃):

Die beiden Multipletts der CH₂-Gruppe des Oxiranrings bei 2,60 - 2,63 ppm und 2,78 - 2,81 ppm sind vollständig verschwunden.

| | | |
|---|---|---|
| 0,95 - 1,01 ppm | (24 H, m) | CH₃ (Piperidin) |
| 1,15 ppm | (24 H, s) | CH₃ (Piperidin) |
| 1,34 - 1,42 ppm | (8 H, m) | CH₂, H axial (Piperidin) |
| 1,83 - 1,89 ppm | (8 H, m) | CH₂, H equatorial (Piperidin) |
| 2,23 ppm | (12 H, s) | N-CH₃ |
| 3,08 - 4,11 ppm | (30 H, m) | keine Zuordnung möglich |
| 6,61 - 6,76 ppm | (4 H, 2d) | Aromat - H |
| 7,01 - 7,03 ppm | (4 H, d) | Aromat - H |

- DSC (N₂, 10°C/Min. Aufheizgeschwindigkeit):: Schmelzpunkt bei 59,6°C
- TGA (Luft, 2°C/Min. Aufheizgeschwindigkeit):: 10 % Gewichtsverlust bei 286°C
- MALDI - MS:: Intensivstes lonensignal bei Masse 1108 Da (protoniertes Molekül), sehr schwache lonensignale bei 894,6, 1029,6, 1322,2 und 1335,6 Da.

Verfährt man nach dem unter B1 beschriebenen Verfahren und setzt als Ausgangsverbindungen die entsprechenden Amine und Epoxide ein, so erhält man die in Tabelle 1 charakterisierten Additionsverbindungen.

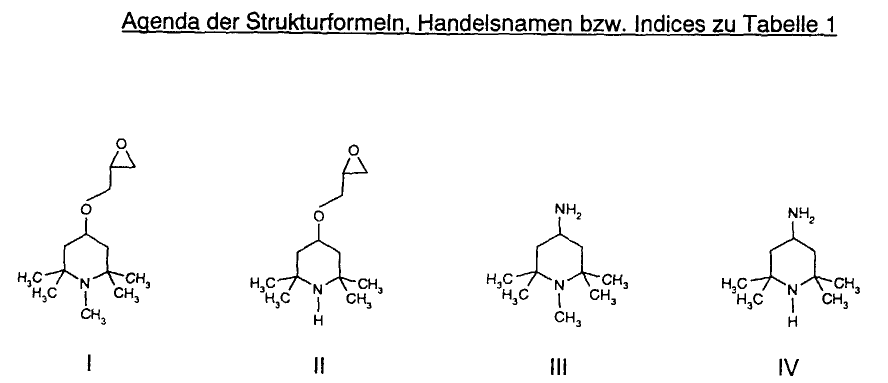

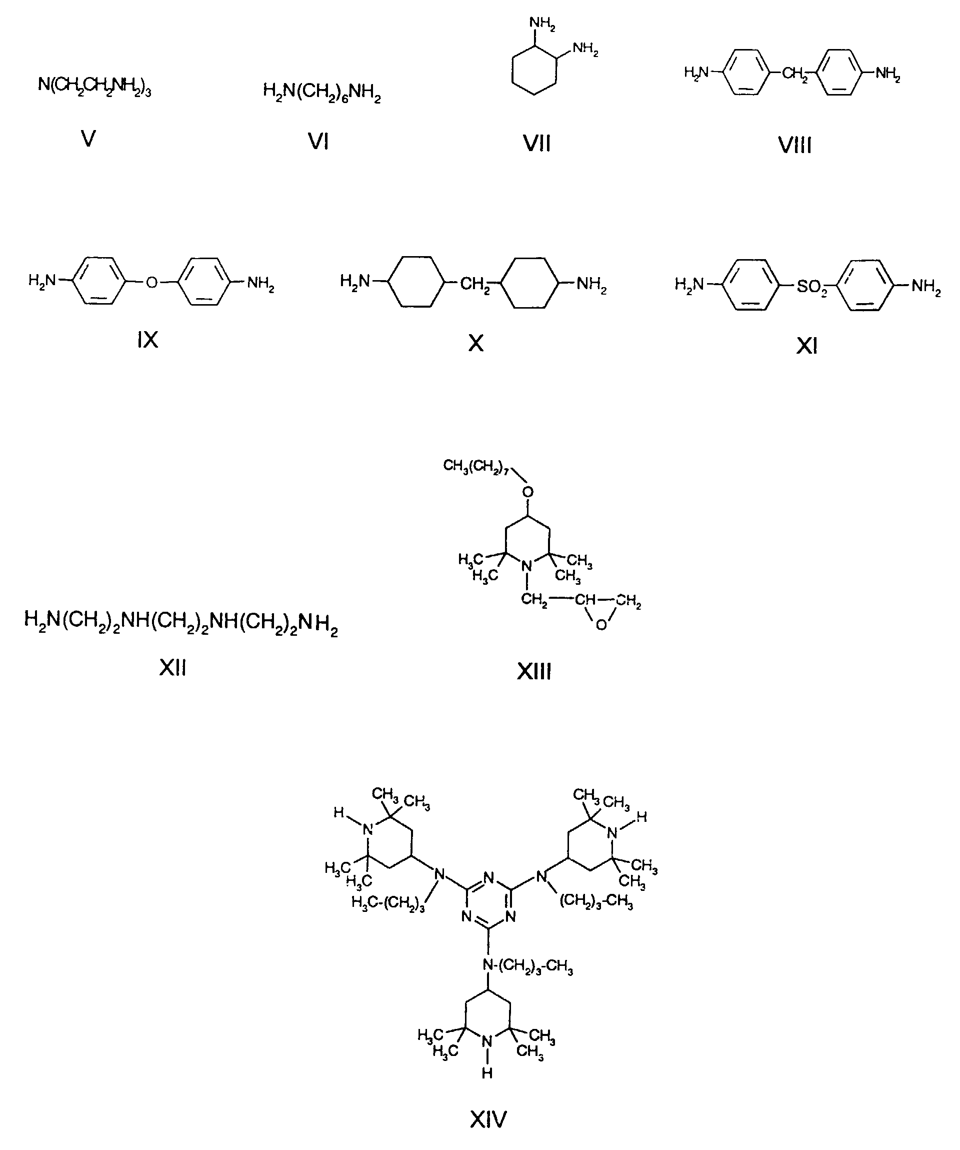

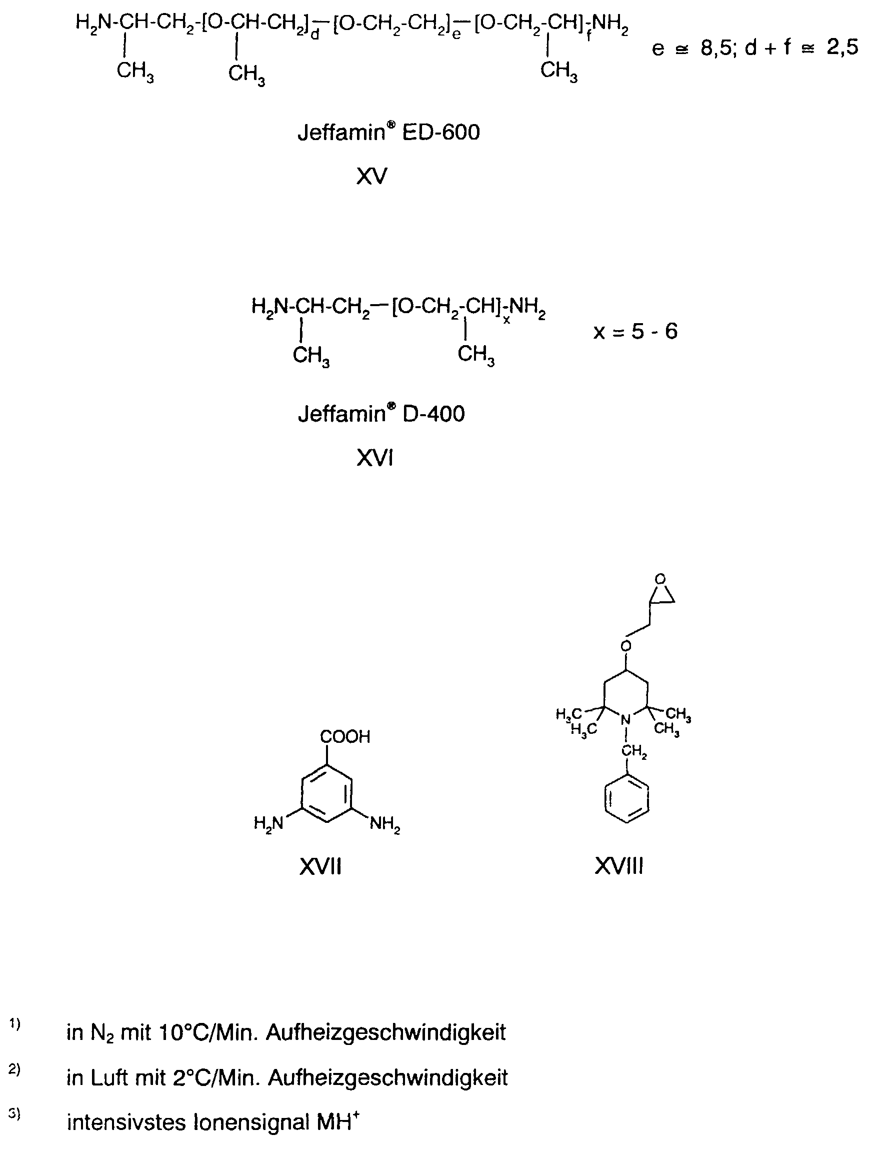

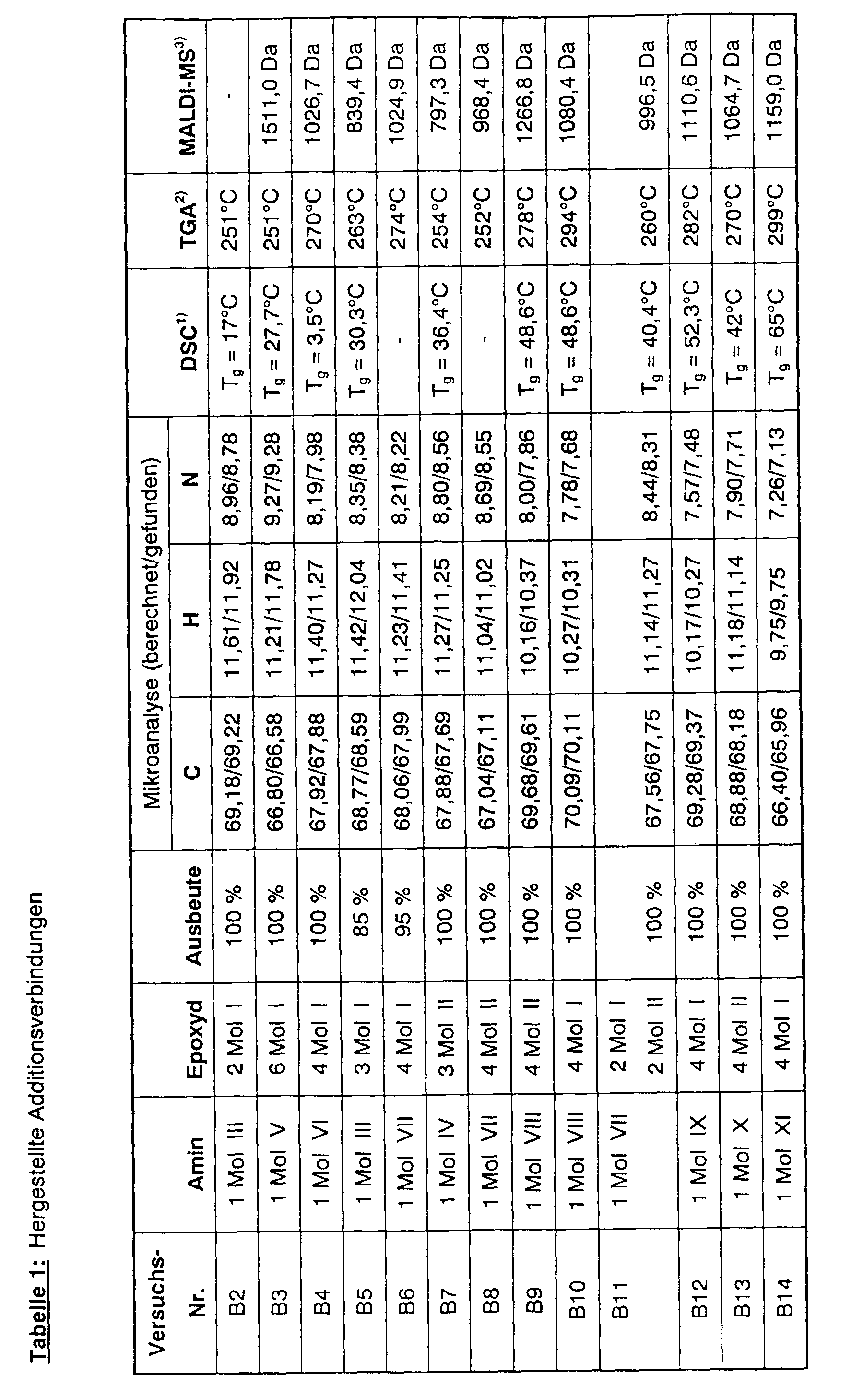

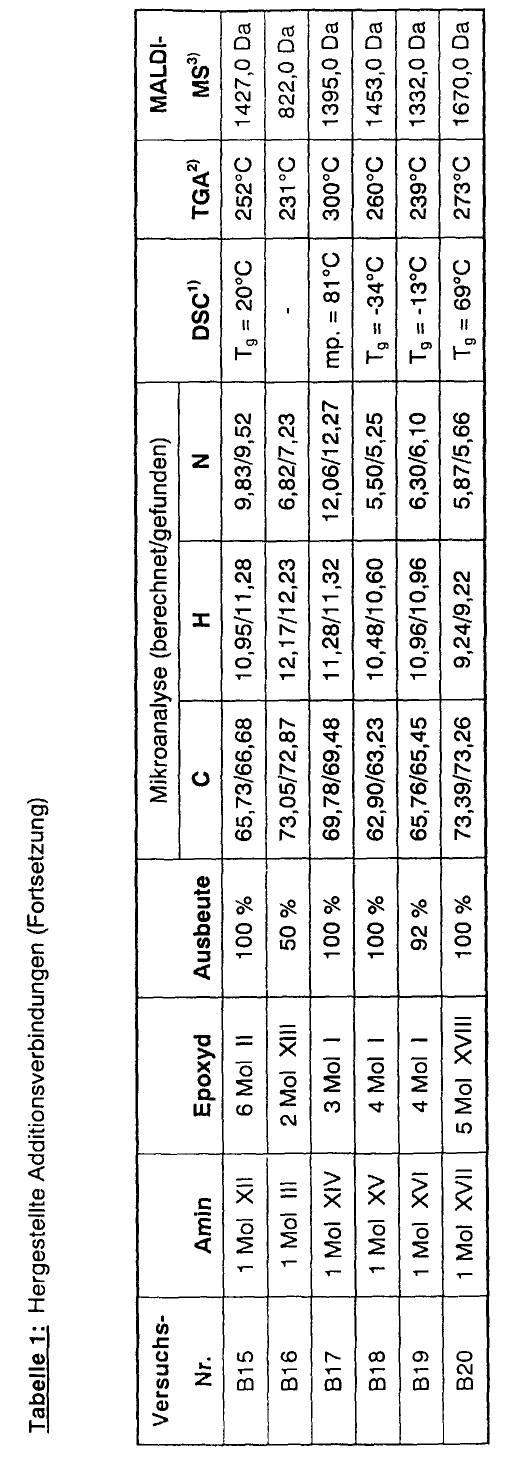

### Beispiel C1: Lichtstabilisierung von Polypropylenfasern

Jeweils 2,5 g des erfindungsgemäßen Stabilisators aus den Beispielen B11 und B14 werden zusammen mit 1 g Tris(2,4-di-tert.butylphenyl)phosphit, 1 g Calcium-Monoethyl-3,5-di-tert.butyl-4-hydroxybenzylphosphonat, 1 g Calciumstearat und 2,5 g TiO₂ (Kronos RN 57) in einem Turbomischer mit 1000 g Polypropylenpulver vermischt (Schmelzindex 12 g/10 min, gemessen bei 230°C/2,16 kg). Die Mischungen werden bei 200-230°C zu Granulat extrudiert; dieses wird anschließend mit Hilfe einer Pilotanlage (Leonard; Sumirago/VA, Italy) unter folgenden Bedingungen zu Fasern verarbeitet:

| | |
|---|---|
| Extrudertemperatur | 190-230°C |
| Kopftemperatur | 255-260°C |
| Streckverhältnis | 1:3,5 |
| Strecktemperatur | 120°C |
| Fasern | 12 den |

Die so hergestellten Fasern werden vor weißem Hintergrund in einem Weather-O-Meter® Typ 65 WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Fasern ohne erfindungsgemäßen Stabilisator unter sonst gleichen Bedingungen hergestellt und getestet. Die Testergebnisse sind in Tabelle 2 zusammengestellt.

**Tabelle 2:**

| Belichtungsdauer bis zur Halbierung der anfänglichen Zugfestigkeit | |
|---|---|
| Stabilisator | Belichtungsdauer |
| ohne | 300 h |
| aus Beispiel B11 | 2860 h |
| Beispiel B14 | 1050 h |

Die erfindungsgemäß stabilisierten Fasern weisen einen hervorragenden Festigkeitserhalt auf.

### Beispiel C2: Stabilisierung eines Zweischicht-Lackes

Die erfindungsgemässen Stabilisatoren aus den Beispielen B2, B3 und B5 werden in 5-10 g Xylol eingearbeitet und in einem Klarlack folgender Zusammensetzung geprüft:

| | |
|---|---|
| Synthacryl® SC 303 ¹⁾ | 27,51 g |
| Synthacryl® SC 370 ²⁾ | 23,34 g |
| Maprenal® MF 650 ³⁾ | 27,29 g |
| Butylacetat/Butanol (37/8) | 4,33 g |
| Isobutanol | 4,87 g |
| Solvesso® 150 ⁴⁾ | 2,72 g |
| Kristallöl K-30 ⁵⁾ | 8,74 g |
| Verlaufshilfsmittel Baysil® MA ⁶⁾ | 1,20 g |
| | 100,00 g |

| | |
|---|---|
| 1) Acrylatharz, Fa. Hoechst AG; 65% Lösung in Xylol/Butanol 26:9 | |
| 2) Acrylatharz, Fa. Hoechst AG; 75% Lösung in Solvesso® 100⁴⁾ | |
| 3) Melaminharz, Fa. Hoechst AG; 55% Lösung in Isobutanol | |
| 4) Hersteller: Fa. ESSO | |
| 5) Hersteller: Fa. Shell | |
| 6) 1% in Solvesso® 150; Hersteller: Fa. Bayer AG | |

Dem Klarlack wird jeweils 1% Stabilisator aus den Beispielen B (in Xylol), bezogen auf den Feststoffgehalt des Lackes, zugesetzt. Einige Klarlackproben enthalten zusätzlich zu den erfindungsgemässen Verbindungen jeweils 1,5 % ®TINUVIN 384:

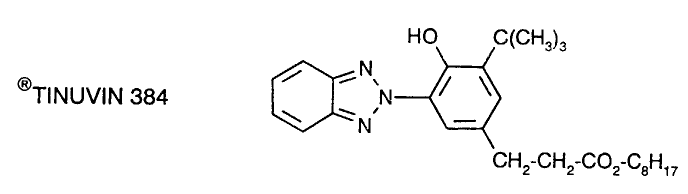

Als Vergleich dient ein Klarlack, der kein Lichtschutzmittel enthält.

Der Klarlack wird mit Solvesso®100 auf Spritzfähigkeit verdünnt und auf ein vorbereitetes Aluminiumblech (coil coat, Füller, silbermetallic Basislack) gespritzt und bei 130°C 30 Minuten eingebrannt. Es resultiert eine Trockenfilmdicke von 40-50 µm Klarlack.

Die Proben werden dann in einem UVCON®-Bewitterungsgerät der Fa. Atlas Corp. (UVB-313 Lampen) bei einem Zyklus von 4 h UV-Bestrahlung bei 60°C und 4 h Kondensation bei 50°C bewittert.

Der Oberflächenglanz (20° Glanz gemäss DIN 67530) der Proben wird in regelmässigen Abständen gemessen.

Die Ergebnisse sind in den Tabellen 2 und 3 zusammengefasst.

**Tabelle 2:**

| Stabilisator | 20° * Glanz gemäss DIN 67530 nach 0, 800, 1600, 2400 und 2800 Stunden Bewitterung im ®UVCON (UVB-313) | | | | |
|---|---|---|---|---|---|
| Stunden | 0 | 800 | 1600 | 2400 | 2800 |
| ohne | 89 | 89 | 11** | | |
| B2 | 89 | 74 | 47 | 3*** | |
| B3 | 89 | 82 | 57 | 40 | 7**** |
| B5 | 89 | 76 | 45 | 2*** | |

| | | | | | |
|---|---|---|---|---|---|
| *: hohe Werte zeigen eine gute Stabilisierungswirkung an | | | | | |
| **: Rissbildung nach 1600 Stunden | | | | | |
| ***: Rissbildung nach 2400 Stunden | | | | | |
| ****: Rissbildung nach 2800 Stunden | | | | | |

**Tabelle 3:**

| Stabilisator | 20° * Glanz gemäss DIN 67530 nach 0, 800, 1600, 2400, 3200 und 3600 Stunden Bewitterung im ®UVCON (UVB-313) | | | | | |
|---|---|---|---|---|---|---|
| Stunden | 0 | 800 | 1600 | 2400 | 3200 | 3600 |
| ohne | 89 | 25** | | | | |
| B2 + ®TINUVIN 384 | 89 | 83 | 81 | 80 | 54 | 23**** |
| B3 + ®TINUVIN 384 | 89 | 87 | 86 | 82 | 50 | 22**** |
| B5 + ®TINUVIN 384 | 89 | 82 | 82 | 73 | 21*** | |

| | | | | | | |
|---|---|---|---|---|---|---|
| *: hohe Werte zeigen eine gute Stabilisierungswirkung an | | | | | | |
| **: Rissbildung nach 800 Stunden | | | | | | |
| ***: Rissbildung nach 3200 Stunden | | | | | | |
| ****: Rissbildung nach 3600 Stunden | | | | | | |

Die den Tabellen 2 und 3 entnehmbaren Ergebnisse zeigen, dass Proben stabilisiert mit den erfindungsgemässen Stabilisatoren (-gemischen) eine bessere Bewitterungsstabilität (geringeren Glanzverlust) haben als nicht stabilisierte Proben.

### Beispiel C3: Stabilisierung von Polypropylen-Platten

1 g jeder der in Tabelle 4 ausgewiesenen Verbindungen, 1 g Tris(2,4-di-tert-butylphenyl)-phosphit, 0,5 g Pentaerythrittetrakis[3-(3,5-di-tert-butyl-4-hydroxyphenyl)proprionat], 1 g Calciumstearat und 1 g ®Filofin Blue G (ex ®Ciba-Geigy) werden in einem Turbomischer mit 1000 g Polypropylenpulver, Schmelzindex = 4 g/10 Min., vermischt (gemessen bei 230°C und 2,16 kg). Die erhaltenen Gemische werden bei einer Temperatur von 200 - 230°C zu Polymergranulat extrudiert und dann zu Platten mit einer Dicke von 2 mm durch Spritzformen bei 200 - 220°C geformt. Die erhaltenen Platten werden in einem Weather-O-Meter Modell 65 Wr (ASTM D2565-85) mit einer Black-Panel-Temperatur von 63°C belichtet, bis Oberflächenbrüchigkeit (Verkreidung) beginnt. Eine unter denselben Bedingungen wie vorstehend ausgewiesen, jedoch unter Zusatz der erfindungsgemässen Verbindungen hergestellte Platte aus Polypropylen wird zum Vergleich belichtet. In Tabelle 4 ist die zu Beginn der Versprödung erforderliche Zeit in Stunden angegeben. Je länger die Zeit, desto besser die stabilisierende Wirkung.

**Tabelle 4:**

| Stabilisator | Verkreidungszeit in Stunden | Stabilisator | Verkreidungszeit in Stunden |
|---|---|---|---|
| ohne | 650 | B8 | 3300 |
| B2 | 3260 | B9 | 2830 |
| B3 | 3260 | B10 | 2400 |
| B4 | 3260 | B11 | 3300 |
| B5 | 3260 | B12 | 2830 |
| B6 | 3260 | B13 | 3300 |
| B7 | 3680 | B14 | 2310 |

Die in Tabelle 4 aufgeführten Ergebnisse zeigen, dass die mit einem erfindungsgemässen Stabilisator stabilisierten Proben eine bessere Verkreidungsstabilität aufweisen als die nicht stabilisierte Probe.

### Beispiel C4: Stabilisierung von Polvpropylen-Bändern

Jeweils 1,0 g des erfindungsgemäßen Stabilisators aus den Beispielen B2 - B14 wird zusammen mit 1 g Tris(2,4-di-tert.butylphenyl)phosphit, 0,5 g Pentaerythrityl-tetrakis(3-[3',5'-di-tert.butyl-4'-hydroxyphenyl]-propionat) und 1 g Calciumstearat in einem Turbomischer mit 1000 g Polypropylenpulver vermischt (STATOILMF; Schmelzindex 4,0 g/10 min, gemessen bei 230°C/2,16 kg).

Die Mischungen werden bei 200-230°C zu Granulat extrudiert; dieses wird anschließend mit Hilfe einer Pilotanlage (Leonard; Sumirago/VA, Italy) unter folgenden Bedingungen zu 2,5 mm breiten Streckbändern von 50 µm Dicke verarbeitet:

| | |
|---|---|
| Extrudertemperatur | 210-230°C |
| Kopftemperatur | 240-260°C |
| Streckverhältnis | 1:6 |
| Strecktemperatur | 110°C |

Die so hergestellten Bänder werden vor weißem Hintergrund in einem Weather-O-Meter® Typ 65 WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Bänder ohne erfindungsgemäßen Stablisator unter sonst gleichen Bedingungen hergestellt und getestet. Die Testergebnisse sind in Tabelle 5 zusammengestellt.

**Tabelle 5:**

| Belichtungsdauer bis zur Halbierung der anfänglichen Zugfestigkeit | |
|---|---|
| Stabilisator | Belichtungsdauer |
| kein | 500 h |
| aus Beispiel B2 | 4770 h |
| aus Beispiel B3 | 4150 h |
| aus Beispiel B4 | 4290 h |
| aus Beispiel B5 | 4640 h |
| aus Beispiel B6 | 4170 h |
| aus Beispiel B7 | 2890 h |
| aus Beispiel B8 | 2730 h |
| aus Beispiel B9 | 1590 h |
| aus Beispiel B10 | 2710 h |
| aus Beispiel B11 | 2600 h |
| aus Beispiel B12 | 1960 h |
| aus Beispiel B13 | 2550 h |
| aus Beispiel B14 | 1360 h |

Die erfindungsgemäß stabilisierte Probe weist einen hervorragenden Festigkeitserhalt auf.

## Patentansprüche

1. Verbindungen erhältlich durch Umsetzung von Verbindungen der allgemeinen Formeln 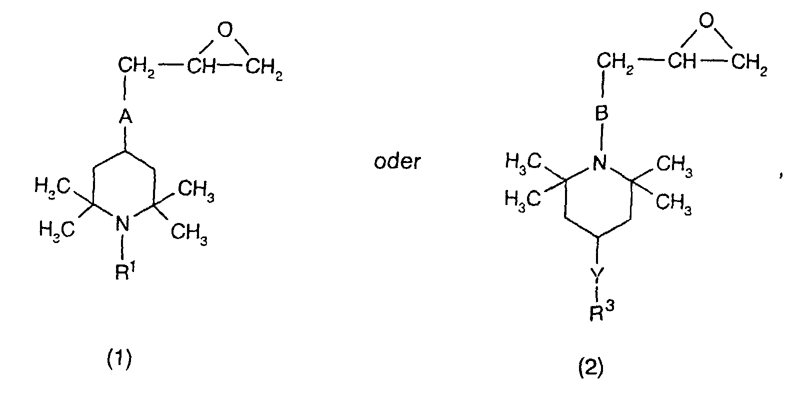 wobei
A gleich O oder N-R² ist und
B eine direkte Bindung oder O-CH₂-CH₂ darstellt, wobei der Kohlenstoff der Ethylengruppe mit dem Stickstoff des Piperidinringes verbunden ist, und
R¹ H, C₁-C₂₀-Alkyl, C₂-C₂₀-Alkenyl, C₂-C₂₀-Alkinyl, C₆-C₂₀-Aryl, C₇-C₂₀-Aralkyl, O-C₁-C₂₀-Alkyl, O-C₅-C₈-Cycloalkyl, CO-C₁-C₂₀-Alkyl, CO-C₆-C₂₀-Aryl, CO-C₇-C₂₀-Aralkyl, O-CO-C₁-C₂₀-Alkyl oder C₁-C₆-Alkyl-Z-C₁-C₆-Alkyl darstellt, wobei
Z O, S oder C=O ist und
R² C₁-C₁₂-Alkyl oder 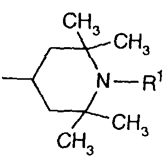 darstellt und
Y O oder N-R⁴ ist, oder Y-R³ nach dem Enffernen des Wasserstoffs in 4-Stellung des Piperidinringes den divalenten Rest 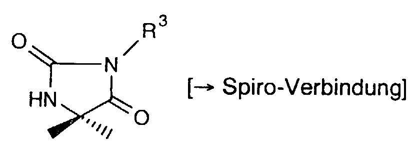 darstellt, und
R³ C₁-C₂₀-Alkyl, CO-C₁-C₂₀-Alkyl, CO-C₆-C₂₀-Aryl oder CO-C₇-C₂₀-Aralkyl bedeuten,
R⁴ C₁-C₂₀-Alkyl, oder, falls
R³ von C₁-C₂₀-Alkyl verschieden ist, auch Wasserstoff bedeutet,
mit mindestens einem sekundären oder primären Amin oder Ammoniak.

2. Verbindungen gemäss Anspruch **1**, worin
R¹ H, C₁-C₁₂-Alkyl, C₂-C₁₂-Alkenyl, C₂-C₁₂-Alkinyl, C₆-C₁₄-Aryl, C₇-C₁₂-Aralkyl, O-C₁-C₁₂-Alkyl, O-C₅-C₈-Cycloalkyl, CO-C₁-C₁₂-Alkyl, CO-C₆-C₁₄-Aryl, CO-C₇-C₁₂-Aralkyl, O-CO-C₁-C₁₂-Alkyl oder C₁-C₄-Alkyl-Z-C₁-C₄-Alkyl darstellt, wobei
Z O, S oder C=O ist und
R² C₁-C₈-Alkyl oder 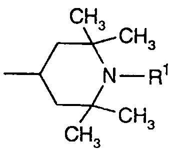 darstellt und
Y O oder N-R⁴ darstellt, und
R³ C₁-C₁₂-Alkyl, CO-C₁-C₁₂-Alkyl, CO-C₆-C₁₄-Aryl oder CO-C₇-C₁₂-Aralkyl bedeutet,
R⁴ C₁-C₁₂-Alkyl, oder, falls
R³ von C₁-C₁₂-Alkyl verschieden ist, auch Wasserstoff bedeutet,
mit mindestens einem sekundären oder primären Amin oder Ammoniak.

3. Verbindungen gemäss Anspruch **1**, wobei
A gleich O ist und
B eine direkte Bindung ist.

4. Verbindungen gemäss Anspruch **1**, wobei
R¹ H, C₁-C₈-Alkyl, C₂-C₈-Alkenyl, C₂-C₈-Alkinyl, C₆-C₁₀-Aryl, C₇-C₁₀-Aralkyl, O-C₁-C₈-Alkyl, O-C₅-C₈-Cycloalkyl oder O-CO-C₁-C₈-Alkyl darstellt, und
Y O und
R³ C₁-C₈-Alkyl, CO-C₁-C₈-Alkyl, CO-C₆-C₁₀-Aryl oder CO-C₇-C₁₀-Aralkyl darstellen.

5. Verbindungen gemäss Anspruch **4**, wobei
R¹ H, CH₃, CH₂-C≡CH, Benzyl, O-C₈-Alkyl, O-Cyclohexyl oder O-CO-CH₃ und
R³ C₈-Alkyl darstellen.

6. Verbindungen gemäss Anspruch **1**, worin die Aminkomponente eine 2,2,6,6-Tetraalkylpiperidinverbindung, ein Alkylamin, Dialkylamin, ein cyclisches Amin, ein Polyalkylenpolyamin, Polyaminoamid, Polyoxyalkylenpolyamin, aromatisches Amin oder nukleophile Aminogruppen enthaltendes Triazin, eine Aminocarbonsäure oder Ammoniak ist.

7. Verbindungen gemäss Anspruch **6**, worin die Aminkomponente ein Piperidinderivat, das mindestens zwei Stickstoffatome enthält, ein Alkylamin, Dialkylamin, Polyalkylenpolyamin, Polyaminoamid, Polyoxyalkylenpolyamin, das aminterminiertes Polyethylenglykol oder Polypropylenglykol oder beide Polyalkylenglykole beinhaltet, aromatisches Amin mit höchstens 2 aromatischen Kohlenstoffringen, an den C-Atomen mit Aminogruppen oder Aminogruppen enthaltenden Substituenten substituiertes Triazin, eine Aminocarbonsäure oder Ammoniak ist.

8. Verbindungen gemäss Anspruch **7**, worin die Aminkomponente ein Piperidin der Formel 3 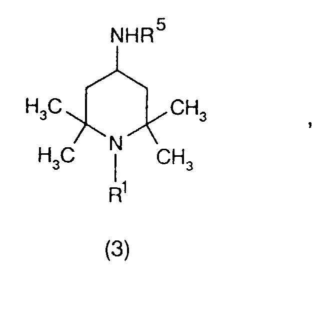 wobei
R¹ H, C₁-C₂₀-Alkyl, C₂-C₂₀-Alkenyl, C₂-C₂₀-Alkinyl, C₆-C₂₀-Aryl, C₇-C₂₀-Aralkyl, O-C₁-C₂₀-Alkyl, O-C₅-C₈-Cydoalkyl, CO-C₁-C₂₀-Alkyl, CO-C₆-C₂₀-Aryl, CO-C₇-C₂₀-Aralkyl, O-CO-C₁-C₂₀-Alkyl oder C₁-C₆-Alkyl-Z-C₁-C₆-Alkyl ist, wobei
Z O, S oder C=O ist und
R⁵ H, C₁-C₁₂-Alkyl oder 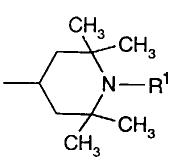 ist;
oder ein Alkylamin oder Dialkylamin, das maximal 20 C-Atome enthält; ein Polyamin der Formeln 4 bis 6 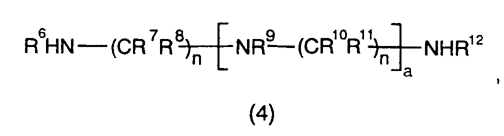 wobei
R⁶ bis R¹² unabhängig voneinander H, CH₃, C₂H₅ oder C₃H₇ sind, und
R⁹ zusätzlich R⁶HN-(CR⁷R⁸)ₙ bedeuten kann,
n 2 bis 20 ist und
a 0 bis 30 ist, 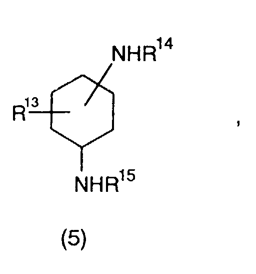 wobei
R¹³ bis R¹⁵ unabhängig voneinander H, CH₃ oder 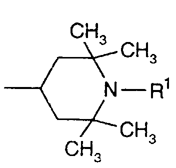 sind, oder 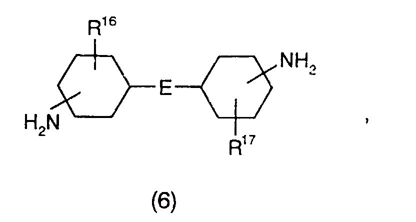 wobei
E eine direkte Bindung oder CH₂, C(CH₃)₂, C=O, N-H, S, SO, SO₂ oder O ist und
R¹⁶ und R¹⁷ unabhängig voneinander H, CH₃ oder 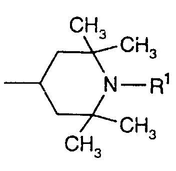 sind;
ein Polyaminoamid der Formel 7 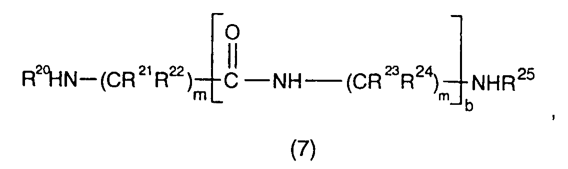 wobei
R²⁰ bis R²⁵ unabhängig voneinander H oder CH₃ sind,
m 1 bis 20 ist und
b 1 bis 30 ist;
ein Polyalkylenpolyamin oder Polyoxyalkylenpolyamin der Formel 8 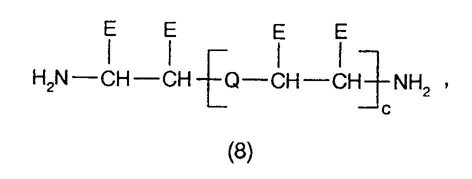 wobei die Symbole
E unabhängig voneinander H oder CH₃ bedeuten und
Q unabhängig voneinander O oder NH ist und
c 1 bis 10.000 ist;
ein aromatisches Amin der Formel 9 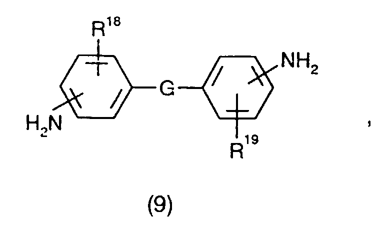 wobei
G eine direkte Bindung oder CH₂, C(CH₃)₂, C=O, N-H, S, SO, SO₂ oder O ist und
R¹⁸ und R¹⁹ unabhängig voneinander H, CH₃ oder C₂H₅ sind;
ein substituiertes Triazin der Formel 10 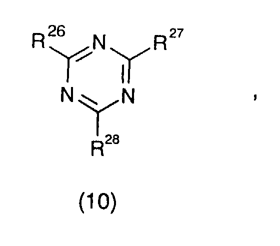 wobei
R²⁶ bis R²⁸ unabhängig voneinander einen primären und/oder sekundären Aminrest enthaltenden Substituenten mit einer C-Zahl von 1 bis 18 darstellen; eine Aminocarbonsäure der Formel 11 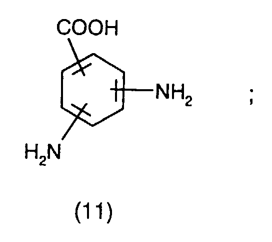 oder Ammoniak ist.

9. Verbindungen gemäss Anspruch **8**, worin in Formel 9 die Aminogruppen in 4,4'-Stellung und R¹⁸ und R¹⁹ in 3,3'-Stellung stehen und R²⁶ bis R²⁸unabhängig voneinander -NH-CH₂-CH₂-NH₂, 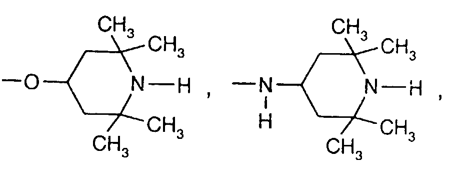 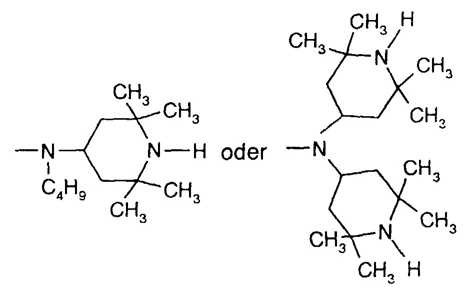 bedeuten.

10. Verbindungen gemäss Anspruch 8, wobei die Amine 4-Amino-2,2,6,6-tetramethylpiperidin, 4-Amino-1,2,2,6,6-pentamethylpiperidin, Tris(2-aminoethyl)amin, Hexamethylendiamin, Triethylentetramin, 1,2-Diaminocyclohexan, 4,4'-Diaminodiphenylmethan, 4,4'-Diaminodiphenylsulfon, 4,4'-Diaminodiphenylether, 4,4'-Diaminodicyclohexylmethan, 3,5-Diaminobenzoesäure, 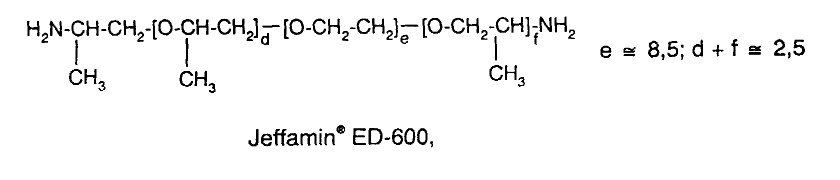 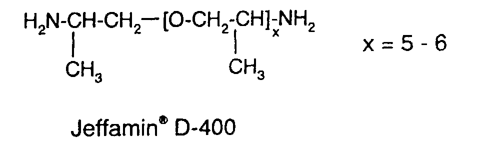 und 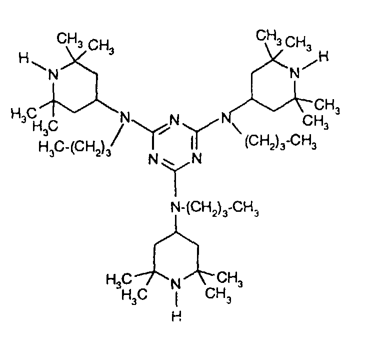 bevorzugt sind.

11. Zusammmensetzung enthaltend a) ein gegen Schädigung durch Licht, Sauerstoff und/oder Wärme empfindliches organisches Material und b) als Stabilisator mindestens eine Verbindung gemäss Anspruch **1**.

12. Zusammensetzung nach Anspruch **11**, worin die Komponente a) eine organisches Polymer ist.

13. Zusammensetzung nach Anspruch **11**, worin die Komponente a) ein synthetisches Polymer ist.

14. Zusammensetzung nach Anspruch **11**, worin die Komponente a) ein Polyolefin oder ein Lackbindemittel auf der Basis von Acryl-, Alkyd-, Polyurethan-, Polyester- oder Polyamidharz oder entsprechend modifizierter Harze ist.

15. Zusammensetzung nach Anspruch **11** enthaltend zusätzlich zu den Komponenten a) und b) weitere übliche Additive.

16. Zusammensetzung nach Anspruch **11** enthaltend 0,01 bis 10 Gew.-% der Komponente b) bezogen auf das Gewicht der Zusammensetzung.

17. Verfahren zur Stabilisierung von organischem Material gegen Schädigung durch Licht, Sauerstoff und/oder Wärme, dadurch gekennzeichnet, dass man diesem Material mindestens eine Verbindung gemäss Anspruch **1** beimischt.

18. Verwendung von Verbindungen gemäss Anspruch **1** zur Stabilisierung von organischem Material gegen Schädigung durch Licht, Sauerstoff und/oder Wärme.
